# EUROPÄISCHE PATENTSCHRIFT

(11) **EP 4 298 427 B1**
(45) Veröffentlichungstag und Bekanntmachung des Hinweises auf die Patenterteilung: **02.07.2025**
(21) Anmeldenummer: 22713541.5
(22) Anmeldetag: 15.02.2022
(51) Int. Cl.: G01N 21/3563, G01N 21/359, G01N 21/84, G01N 33/46

(54) **VERFAHREN ZUR BESTIMMUNG DER HARZPENETRATION IN HOLZ MITTELS NAH-INFRAROT-SPEKTROSKOPIE**
METHOD OF DETERMINING RESIN PENETRATION IN WOOD USING NEAR INFRARED SPECTROSCOPY
PROCÉDÉ DE DÉTERMINATION DE LA PÉNÉTRATION DE RÉSINE DANS BOIS UTLISANT LA SPECTROSCOPIE INFRAROUGE PROCHE

(30) Priorität: 24.02.2021 EP 21158965
(43) Veröffentlichungstag der Anmeldung: 03.01.2024
(73) Patentinhaber: Flooring Technologies Ltd., Kalkara SCM1001 (MT)
(72) Erfinder: HASCH, Joachim, 10317 Berlin (DE); KALWA, Norbert, 32805 Horn-Bad Meinberg (DE); ZHANG, Jinming, 10247 Berlin (DE)
(74) Vertreter: Maikowski & Ninnemann Patentanwälte Partnerschaft mbB
(86) Internationale Anmeldenummer: PCT/EP2022/053620
(87) Internationale Veröffentlichungsnummer: WO 2022/179883

(56) Entgegenhaltungen:
- EP-A1- 3 327 424
- WO-A1-2007/021235
- CN-A- 105 334 179

## Beschreibung

Die vorliegende Erfindung betrifft ein Verfahren zur Bestimmung der Harzpenetration in mindestens ein poröses Beschichtungsmaterial, welche mit mindestens einer Trägerplatte und mindestens einer auf der Trägerplatte angeordneten Harzschicht verpresst ist, wobei während des Pressvorganges das Harz in das mindestens eine poröse Beschichtungsmaterial penetriert bzw. aufsteigt.

### Beschreibung

Bodenbeläge werden zunehmend mit verschiedenen Beschichtungsmaterialien wie Leder, Filz oder Echtholz versehen, um gestiegene Kundenwünsche zu erfüllen.

Bei der Herstellung von Bodenbelägen mit einer Echtholzoberfläche werden verschiedene Technologien eingesetzt. Ein Ansatz besteht darin relativ dicke Echtholzfurniere bis zu einer Stärke von mehreren Millimetern auf quer dazu angeordnete Holzlagen aufzuleimen. Als Unterlage werden quer zu der Mittellage liegenden Holzschichten eingesetzt.

Ein anderer Ansatz besteht darin, die Holzlagen durch Holzwerkstoffe zu ersetzen. Somit wird in hierbei ein Echtholzfurnier auf einen Holzwerkstoffträger (HDF, Spanplatte, OSB usw.) aufgeleimt. Die hierbei verwendeten Furniere weisen typischerweise eine geringere Dicke auf, was eine geringere mechanische Festigkeit bedingt, als relativ hohe Furnierstärken.

Der Vorteil der Verwendung von dünnen Echtholzfurnieren besteht in den günstigeren Produktions- und Materialkosten. Allerdings erfordert die Verwendung von Furnieren eine geeignete Oberflächenvergütung. Eine mögliche Oberflächenvergütung besteht dabei üblicherweise aus einer Lackierung auf Basis von UV- oder ESH-Lacken.

Zur Verklebung der Furniere auf dem Träger werden meist Harnstoff- oder PVAc-Leime mit Härter eingesetzt. Auf der Rückseite des Produktes befindet sich meist ein Gegenzug auf Basis von Furnier, der die Spannungssymmetrie im Produkt gewährleisten soll. Zudem wird dadurch auch der Aspekt eines Holzfußbodens zusätzlich unterstützt.

Ein Problem stellt bei diesem Produkt die nur eingeschränkte Möglichkeit dar, das Produkt bei Beschädigung zu reparieren. Dies ist umso gravierender, weil die mechanische Stabilität der Furnierlage wegen der geringen Rohdichte des Furniers (300 - 500 kg/m³) nicht besonders hoch ist. Bei mechanischer Beschädigung durch z. B. herabfallende Gegenstände werden bei einem derartigen Produkt also schnell tiefe Eindrücke erzeugt.

Eine Lösung für dieses Problem liefert die WO 2015/105456 A1, bei der ein Gemisch von Holzmehl und Melaminharzpulver auf eine Holzwerkstoffplatte aufgestreut wird und anschließend zusammen mit einem Furnier auf die Holzwerkstoffplatte aufgepresst wird. Hier wird ein möglichst weitgehendes Penetrieren des Melaminharzes gewünscht, eine direkte Kontrolle der Penetrationshöhe des Harzes in das Furnier ist allerdings nicht möglich.

CN105334179 A offenbart ein Verfahren zur Bestimmung der Eindringtiefe von Holzkleber in Holzproben mittels Fourier-Transformierte Infrarot (FTIR) - Mikrospektroskopie. Für einen ausgewählten Bildausschnitt wird ein Infrarotbild erstellt, wobei hierfür nur der charakteristische Wellenlängenbereich des Holzklebers zwischen 1640-1660cm-1 verwendet wird. In diesem Infrarotbild ist ein Hell-Dunkel-Kontrast zu erkennen, in welchem der gemessene helle Anteil der Eindringtiefe des Klebers entspricht. Aus den hellen Anteilen des mikroskopischen IR-Bildes wird die Eindringtiefe ermittelt.

Aus der WO 2007/021235 ist ein Kalibrationsmodell zur Bestimmung der erforderlichen Harzmenge beim Verpressen zweier Schichten bekannt. Das Kalibrationsmodell wird zur Auswertung von NIR-Spektren eingesetzt, und berücksichtigt unter anderem die Eindringtiefe von Harz in die Trägerplatte.

Die oben bezüglich der verleimten Furniere geschilderten Probleme werden ebenfalls durch eine neue Technologie im Wesentlichen behoben. Dabei wird das Furnier in einer Kurztaktpresse mit Hilfe eines mit Melaminharz imprägnierten Papiers (z. B. ein Overlay ) auf den Holzwerkstoffträger aufgepresst. Die Pressparameter liegen dabei bei ca. T > 150°C, p > 30 bar und t > 30 sec. Mit dieser Technologie lassen sich ebenfalls Furnierfussböden mit Furnieren herstellen, die eine Stärke von ca. 0,5 mm besitzen. Von entscheidender Bedeutung ist dabei, dass auch hier beim Presssvorgang das Melaminharz möglichst weit in das Furnier aufsteigt. Dadurch erfolgt zum einen eine Armierung des Furniers mit dem Kunstharz und zum anderen wird das durch die Verpressung komprimierte Furnier in dem Zustand fixiert. Allerdings sollte das Melaminharz nicht aus dem Furnier austreten, da dies zu Verfärbungen in der Oberfläche und zu Haftungsproblemen beim anschließenden Lackieren oder Ölen führt. Ein Problem ist nun, dass die Bestimmung der Güte der Armierung (d.h. das Aufsteigen des Melaminharzes in das Furnier) nicht zerstörungsfrei bzw. on-line durchgeführt werden kann. Dies ist umso gravierender, da je nach Kollektion bzw. Nutzungsklasse sowohl unterschiedliche Holzfurniere als auch unterschiedliche Furnierstärken verarbeitet werden. Zudem können sich auch Furniere gleicher Holzart aus unterschiedlichen Regionen bezüglich ihrer Eigenschaften unterscheiden.

Es ergeben sich somit folgende Nachteile: keine zerstörungsfreie Prüfung des Prozesses möglich; höhere Kosten durch Qualitätsbestimmung und Nachjustierungen an Pressparameter notwendig.

Der Erfindung liegt daher die technische Aufgabe zu Grunde eine zerstörungsfreie Methode zu entwickeln, die eine Bestimmung des Grades der Harzpenetration in poröse Beschichtungsmaterialien, wie Furniere ermöglicht. Die Methode sollte möglichst schnell Ergebnisse liefern, damit in der Produktion möglichst geringe oder gar keine Stillstandzeiten wegen der Qualitätsbestimmung entstehen. Die Harzpenetration soll bereits unmittelbar hinter der Presse möglich sein und eine kontinuierliche Überwachung dieses Parameters ermöglichen.

Diese Aufgabe wird durch ein Verfahren mit den Merkmalen des Anspruchs 1 gelöst.

Demnach wird ein Verfahren zur Bestimmung der Harzpenetration (Penetrationshöhe des Harzes) in mindestens ein poröses Beschichtungsmaterial bereitgestellt, wobei das mindestens eine poröse Beschichtungsmaterial mit mindestens einer Trägerplatte und mindestens einer auf der Trägerplatte angeordneten Harzschicht verpresst ist, und wobei während des Pressvorganges das Harz in das mindestens eine poröse Beschichtungsmaterial penetriert bzw. aufsteigt. Das vorliegende Verfahren umfasst die folgenden Schritte:
- Aufnahme von mindestens einem NIR-Spektrum von mehreren Referenzproben mit jeweils unterschiedlichem Werten für die Harzpenetration in ein poröses Beschichtungsmaterial unter Verwendung von mindestens einem NIR-Messkopf in einem Wellenlängenbereich zwischen 500 nm und 2500 nm, bevorzugt zwischen 700 nm und 2000 nm, insbesondere bevorzugt zwischen 900 nm und 1700 nm, und besonders vorteilhaft zwischen 1450 nm und 1550 nm;
- Bestimmung der Harzpenetration in das poröse Beschichtungsmaterial der genannten Referenzproben mittels eines mechanischen Abtrages der porösen Materialoberfläche;
- Zuordnung der mittels mechanischen Abtrages bestimmten Harzpenetration zu den aufgenommenen NIR-Spektren der genannten Referenzproben; und
- Erstellung eines Kalibriermodells für den Zusammenhang zwischen den spektralen Daten der NIR-Spektren und den dazugehörigen Harzpenetrationen der Referenzproben mittels einer multivariaten Datenanalyse;
- Verpressen von mindestens einem porösem Beschichtungsmaterial mit mindestens einer Trägerplatte und mindestens einer auf der Trägerplatte angeordneten Harzschicht,
- Aufnehmen von mindestens einem NIR-Spektrum der mit der Trägerplatte und der Harzschicht verpressten porösem Beschichtungsmaterial unter Verwendung des mindestens einen NIR-Messkopfes in einem Wellenlängenbereich zwischen 500 nm und 2500 nm, bevorzugt zwischen 700 nm und 2000 nm, insbesondere bevorzugt zwischen 900 nm und 1700 nm und besonders vorteilhaft zwischen 1450 nm und 1550 nm; und
- Bestimmen der Harzpenetration in das mindestens eine poröse Beschichtungsmaterial durch Vergleich des für das poröse Beschichtungsmaterial aufgenommenen NIR-Spektrums mit dem erstellten Kalibriermodell.

Gemäß dem vorliegenden Verfahren wird ein NIR-Spektrum der porösen Materialoberfläche aufgenommen. Es wird eine NIR-Strahlung erzeugt und auf die zu analysierende Trägermaterial-Probe mit der Materialoberfläche geleitet, wo die NIR-Strahlung mit den Bestandteilen der Probe wechselwirkt und reflektiert bzw. gestreut wird. Ein NIR-Detektor fängt die reflektierte bzw. gestreute NIR-Strahlung auf und erzeugt ein NIR-Spektrum, das die gewünschten chemischen Informationen der Probe beinhaltet. Bei dieser Messung wird in einer Sekunde eine Vielzahl von einzelnen NIR-Messungen durchgeführt, sodass auch eine statistische Absicherung der gemessenen Werte gewährleistet wird. Die NIR-Spektroskopie zusammen mit der (unten angeführten) Multivariaten Datenanalyse bietet eine Möglichkeit an, einen direkten Bezug zwischen den spektralen Informationen (NIR-Spektren) und den zu bestimmenden Parametern desaufgebrachten porösen Beschichtungsmaterials, wie z.B. einer Furnierlage herzustellen.

Das vorliegende Verfahren nutzt den Umstand aus, dass die NIR-Strahlung nicht durch das Trägermaterial hindurch dringt, sondern an der Oberfläche der Trägerplatte reflektiert bzw. gestreut wird. Die reflektierte bzw. gestreute NIR-Strahlung wird von dem NIR-Detektor erfasst, und das ermittelte NIR-Spektrum wird zur Bestimmung der gewünschten Parameter (hier Penetrationshöhe des Harzes in das Beschichtungsmaterial) verwendet.

Das aufgenommene NIR-Spektrum ermöglich es in Kombination mit einer Prüfung des Prozentsatzes der Penetration über ein mechanisches Abtragen der porösen Materialoberfläche eine Korrelation zu erzeugen. Es hat sich nämlich überraschenderweise gezeigt, dass je nachdem wie weit das Harz z.B. Melaminharz in das poröse Beschichtungsmaterial aufsteigt, eine Signalerhöhung für den Melaminpeak zu beobachten ist.

Es werden zunächst Referenzproben des einer mit einem porösen Beschichtungsmaterial und Harzschicht verpressten Trägerplatte bereitgestellt. Wesentlich ist, dass die Referenzprobe gleichartig zu der zu vermessenden Probe ist; d.h. insbesondere die Harzschicht und poröses Beschichtungsmaterial der Referenzprobe weisen die gleiche Zusammensetzung wie die zu vermessende Harzschicht und poröses Beschichtungsmaterialauf. Die Gleichartigkeit von zu vermessender Probe und Referenzprobe ist insbesondere bei Verwendung von Harzschichten mit Zusatzstoffen wie Flammschutzmitteln, Fasern, weiteren Additiven wesentlich.

Von diesen Referenzproben werden zumindest ein NIR-Spektrum in einem Wellenlängenbereich zwischen 500 nm und 2500 nm, bevorzugt zwischen 700 nm und 2000 nm, insbesondere bevorzugt zwischen 900 nm und 1700 nm aufgenommen.

Diese Referenzproben werden ebenfalls einer nicht-spektroskopischen Analyse zur Bestimmung der gewünschten Parameter, d.h. im vorliegendem Fall eines mechanischen Abtrages der porösen Materialoberfläche, zugeführt.

Aus den jeweils mittels der nicht-spektroskopischen Analyse ermittelten Parameter für die Referenzproben wird ein Mittelwert gebildet, welcher dann den jeweils aufgenommen NIR-Spektren dieser Referenzproben zugeordnet wird, und es wird ein Kalibriermodell für den Zusammenhang zwischen den spektralen Daten der NIR-Spektren der Referenzproben und den dazugehörigen Parameterwerten mittels einer multivariaten Datenanalyse erstellt; d.h. zu jedem Parameterwert der Referenzprobe korrespondiert ein NIR-Spektrum der Referenzprobe. Die für die verschiedenen Parameter erstellten Kalibriermodelle werden in einem geeigneten Datenspeicher hinterlegt.

Anschließend wird mindestens ein poröses Beschichtungsmaterial mit Harzschicht und Trägerplatte verpresst und mindestens ein NIR-Spektrum des verpressten poröses Beschichtungsmaterials aufgenommen. Der gewünschte Parameter des porösen Beschichtungsmaterials (hier die Harzpenetration bzw. Penetrationshöhe in das poröse Beschichtungsmaterial) kann dann durch Vergleich des für das verpresste poröses Beschichtungsmaterialaufgenommenen NIR-Spektrums mit dem erstellten Kalibriermodell bestimmt werden.

Es ist somit möglich, aus einem einzigen für die zu vermessende Probe ermittelten NIR-Spektrum durch einen automatisierten Vergleich bzw. Abgleich mit den für die jeweiligen Parameter erstellten Kalibriermodellen gleichzeitig mehrere interessierende Parameter des mit der Trägerplatte verpressten porösen Beschichtungsmaterial zu bestimmen.

Ein Vergleich und die Interpretation der NIR-Spektren erfolgt sinnvollerweise über den gesamten aufgenommenen Spektralbereich. Dies wird vorteilhaft mit einer an sich bekannten multivariaten Datenanalyse (MDA) durchgeführt. Bei multivariaten Analysemethoden werden in an sich bekannter Weise typischerweise mehrere statistische Variablen zugleich untersucht. Hierzu wird bei diesen Methoden üblicherweise die in einem Datensatz enthaltene Zahl von Variablen reduziert, ohne gleichzeitig die darin enthaltene Information zu mindern.

Im vorliegenden Fall erfolgt die multivariate Datenanalyse über das Verfahren der Partial Least Squares Regression (partielle Regression der kleinsten Quadrate, PLS) wodurch ein geeignetes Kalibrationsmodell erstellt werden kann. Die Auswertung der gewonnenen Daten wird bevorzugt mit einer geeigneten Analysesoftware vorgenommen wie z.B. mit der Analysesoftware SIMCA-P der Firma Umetrics AB oder The Unscrambler der Firma CAMO. In einer weiteren Ausführungsform ist vorgesehen, für die Erstellung des Kalibriermodells spektrale Daten aus dem NIR- Spektralbereich zwischen 1450 und 1550 nm zu verwenden, die mittels geeigneter mathematischer Methoden vorbehandelt werden und anschließend der multivariaten Datenanalyse zugeführt werden.

Die Bedeutung einer Wellenlänge für die Vorhersage von Parametern des verpressten porösen Beschichtungsmaterials, wie z.B. der Harzpenetration, aus dem NIR-Spektrum wird mit Hilfe der Regressionskoeffizienten dargestellt. Dabei haben die Regionen mit großen Koeffizientenbeträgen starken Einfluss auf das Regressionsmodell. So zeigt die Darstellung der Regressionskoeffizienten in einem PLS-Regressionsmodell für die Bestimmung der Harzmenge, welches nicht unter den beanspruchten Gegenstand fällt, dass der Wellenlängenbereich zwischen 1460 nm und 1530 mit Maximum bei 1490 nm (Absorptionsbande der Amino-Gruppen des Harzes) für die Berechnung des Modells am wichtigsten ist, da hier die Beträge der Regressionskoeffizienten am größten sind. Die anderen Bereiche im Spektrum haben zwar geringeren Informationsgehalt in Bezug auf die NIR-Messung, tragen aber dennoch dazu bei, die weiteren Informationen bzw. störenden Einflussgrößen (wie Transparenz der Schicht, Oberflächenbeschaffenheit der Harzschicht oder des Trägermaterials usw.) zu berücksichtigen bzw. zu minimieren.

Zur Eliminierung von störenden Einflüssen (wie z.B. Beschaffenheit der Oberfläche des Trägermaterials oder des porösen Beschichtungsmaterials, Farbigkeit der Proben, Lichtstreuung an Feststoffpartikeln oder anderen Additiven usw.) ist es notwendig, die spektralen Daten mit mathematischen Vorbehandlungsmethoden (z. B. derivative Datenvorbehandlung, Standardisierung gemäß SNVT (Standard Normal Variate Transformation), multiplikative Signal-Korrektur (EMSC, Extended Multiplicative Signal Correction usw.) zu bearbeiten. Dabei werden die Basislinieneffekte, die hauptsächlich durch die unterschiedliche Farbe der Proben verursacht werden, aus den Spektren entfernt, überlagernde Banden voneinander getrennt und die Abhängigkeit der Lichtstreuung an der Substratoberfläche berücksichtigt. So erfolgt die Datenvorbehandlung bevorzugt zur Reduzierung der Lichtstreuung an der rauen Oberfläche des Substrates. Bei der Messung liegt der Schwerpunkt der Kalibrierung und Datenvorbehandlung auf der Entfernung der Basislinienverschiebung.

Aus den vorbehandelten Daten wird mit Hilfe der multivariaten Datenanalyse ein Kalibriermodell entwickelt, das alle bei der Kalibrierung verwendeten Dekore beinhaltet.

Entsprechend erfolgen der Vergleich und die Interpretation der NIR-Spektren bevorzugt im Spektralbereich zwischen 1450 und 1550 nm unter Verwendung der multivariaten Datenanalyse MDA. Bei multivariaten Analysemethoden werden in an sich bekannter Weise typischerweise mehrere statistische Variablen zugleich untersucht. Hierzu wird die in einem Datensatz enthaltene Zahl von Variablen reduziert, ohne gleichzeitig die darin enthaltene Information zu mindern.

Im vorliegenden Verfahren wurden für die Erstellung der Korrelation Reihenverpressungen durchgeführt, bei denen eine HDF (Faserplatte mit erhöhter Rohdichte) mit unterschiedlichen Mengen flüssigem und anschließend getrocknetem Melaminharz bestrichen wurde. Dann wurden auf diese HDF ein poröses Beschichtungsmaterial, z.B. ein Eichenfurniermit einer Stärke von 0,5 mm, aufgelegt und verpresst. Von diesen Proben wurden NIR-Spektren angefertigt. Dabei zeigte sich, dass der Melaminharzpeak, je nach Penetration mehr oder weniger deutlich ausgeprägt war. Durch einen mechanischen Abtrag des porösen Materials wurde dann die Penetrationshöhe des Melaminharzes in das poröse Material bestimmt und mit den NIR-Spektren in Beziehung gesetzt. Dabei kann zur besseren Sichtbarmachung des Harzes bzw. der Harzfront eine Einfärbung des Harzes vorgenommen werden, die aber die NIR-Spektroskopie nicht stört.

Bei dem Verpressen (Heißpressen) bewirken Druck und Temperatur die Polykondensationsreaktion, in das Harz, insbesondere Melaminharz aushärtet. Dazu wird das Harz vom Zustand b (teilweise kondensiert, noch schmelzbar und härtbar) in den Zustand c (vollständig kondensiert und ausgehärtet) überführt. Dazwischen ist es flüssig und dies wird ausgenutzt, um das Aufsteigen zu ermöglichen und hier mit NIR zu betrachten/bewerten.

Das vorliegende Verfahren ermöglicht die Bereitstellung der Messwerte in kurzer Zeit (online, bevorzugt ohne störende Zeitverzögerung) im Vergleich zu herkömmlichen (bekannten) Messverfahren. Die Messdaten können zur Qualitätssicherung, Forschung und Entwicklung, zur Prozesskontrolle, -regelung, -steuerung usw. eingesetzt werden. Durch den Messvorgang wird die Produktionsgeschwindigkeit usw. nicht reduziert. Grundsätzlich wird damit die Überwachung der Produktion verbessert. Zudem werden auch Stillstandszeiten durch Qualitätsbestimmungen und Anlagenjustierungen reduziert.

Die Vorteile des vorliegenden Verfahrens sind vielfältig: Berührungslose Multiparameterbestimmung ("real time"- oder "Echtzeit"-Messung) mit deutlich reduzierter zeitlicher Verzögerung in der Auswertung der gemessenen Parameterwerte; verbesserte Anlagensteuerung bzw. -regelung, Verringerung des Ausschusses, Verbesserung der Qualität der auf der Anlage hergestellten Produkte, Verbesserung der Anlagenverfügbarkeit.

In einer Ausführungsform des vorliegenden Verfahrens umfasst die mindestens eine Harzschicht eine harzgetränkte Papierlage, ein harzhaltiges Pulver oder eine harzhaltige Flüssigkeit. Die aufgetragene Harzschicht kann demnach als Pulver- oder Flüssigoverlay oder als Teil- oder Vollimprägnat einer Papierlage auf der Trägerplatte vorliegen.

### Harzgetränkte Papierlage (Overlay)

Die harzgetränkte Papierlage basiert typischerweise auf einer zellulosehaltigen Lage mit einem durchschnittlichen Blattgewicht von 18-50 g/m², bevorzugt 20-30 g/m², z.B. 25 g/m².

Derartige zellulosehaltige Lagen sind mit duroplastischen Harzen als Bindemittel imprägniert, wie zum Beispiel Formaldehydharze, insbesondere Melaminharz, Phenolharz, Harnstoffharz oder Mischungen dieser Harze. Solche Papierlagen sind auch als Overlay bekannt.

Zur Imprägnierung der Papierlagen werden wässrige Harzlösungen mit einem Harzfeststoffgehalt zwischen 40 und 80 Gew%, bevorzugt zwischen 50 und 65 Gew% eingesetzt.

Das Harz wird in einer Menge von 200 % bis 600%, bevorzugt 250 % bis 400 % Feststoffgehalt bezogen auf das Flächengewicht der Papierlage aufgebracht. Das Harz wird dabei in einer Menge eingesetzt, die die ausreichend ist, so dass das Harz das poröse Beschichtungsmaterial während des Pressvorganges mindestens abschnittsweise durchdringen kann.

Zur Imprägnierung wird die Papierlage als Rollenware von einer Abwickelstation abgerollt, durch ein Tränkbad mit flüssigem Harz gezogen und imprägniert. Danach folgt eine Trocknung in einem Schwebetrockner und eine Aufwicklung oder Formatierung des harzgetränkten Papiers.

In einer bevorzugten Ausführungsform ist es möglich nach dem Imprägnierschritt auf die noch nicht vorgetrocknete und daher noch feuchte Papierlage Harz als Feststoff (z.B. als Pulver, Staub, Granula) aufzubringen. Dies kann zum Beispiel durch Sprühen mittels Tribopistolen erfolgen. Die aufgetragenen Harzmengen, z.B. in Form von Melaminharzpulver, können zwischen 10 und 50 g/m², bevorzugt zwischen 15 und 30 g/m² betragen. Die Menge des auf die Papierlage aufgebrachten festen Harzes z.B. in Form von Melaminharzpulver wird durch die zu erreichende Penetrationshöhe des Harzes in dem porösen Beschichtungsmaterial bestimmt.

Nach dem Aufbringen des Harzes auf die Papierlage, insbesondere nach dem Imprägnieren der Papierlage mit dem Harz, ist die Oberfläche lediglich vorgetrocknet und somit noch klebrig. Dieser klebrige Zustand wird bei einem Gehalt an flüchtigen Substanzen mit einer Restfeuchte (VC-Wert) von 10-15 % erreicht. Der VC-Wert wird als Differenz zwischen Ausgangsgewicht und Endgewicht nach Trocknen bei 105 °C bis zur Gewichtskonstanz bestimmt.

Die klebrige Oberfläche der harzgetränkten Papierlage vereinfacht das Aufbringen von Additiven zur weiteren Veredelung des porösen Beschichtungsmaterials, wie z.B. einer Furnierlage.

### Pulverförmiges Harz (Pulveroverlay)

Im Falle der Verwendung von pulverförmigem Harz beträgt die Menge des auf die Oberfläche der Trägerplatte aufgetragenen pulverförmigen Harzes 50-150g/m², bevorzugt 60-100 g/m², insbesondere bevorzugt 70-80 g/m². Die Menge an verwendetem Harz ergibt sich aus den Verklebungseigenschaften und aus der gewünschten Penetrationshöhe des Harzes in das poröse Beschichtungsmaterial.

Das zum Einsatz kommende pulverförmige Harz weist eine Streudichte von 0,5 bis 1,5 kg/l, bevorzugt 0,8 bis 1,0 kg/l und eine mittlere Partikelgröße von 10 bis 50 µm, bevorzugt 20 bis 30 µm, insbesondere bevorzugt von 25 µm hat.

Das vorliegend verwendete pulverförmige Harz weist nur geringe Spuren von Feuchtigkeit auf. So sollte eine Feuchte von 0,5% nicht überschritten werden, da es ansonsten zu einer Klumpenbildung kommt und ein Streuen nicht mehr möglich ist.

In einer weitergehenden Variante des vorliegenden Verfahrens wird die mit dem pulverförmigen Harz zu bestreuende Oberfläche bzw. Seite der Trägerplatte vor dem Aufstreuen des pulverförmigen Harzes zur Verbesserung der Haftung des pulverförmigen Harzes auf der Oberfläche der Trägerplatte vorbehandelt. Diese Vorbehandlung kann eine Beaufschlagung der Seite bzw. Oberfläche mit Feuchtigkeit oder eine elektrostatische Aufladung der Seite bzw. Oberfläche der Trägerplatte umfassen.

Als pulverförmiges Harz wird ein Formaldehydharz, bevorzugt ein Harnstoffharz, ein Melaminharz oder ein Phenolharz, insbesondere bevorzugt ein Melamin-Formaldehyd-Harz, ein Melamin-Phenol-Formaldehyd-Harz oder ein Melamin-Harnstoff-Formaldehydharz verwendet.

Das pulverförmige Harz wird bevorzugt unter Verwendung einer Streuapparatur aufgebracht. Das Aufstreuen erfolgt bevorzugt in einem kontinuierlichen Durchlaufprozess. Eine geeignete Streuapparatur ist der Präzisionsstreuer "Oszillierendes Ausbürstsystem" der Fa. TPS. Es kann aber auch eine elektrostatische Applikation mit einer Tribopistole erfolgen.

Diese weitere aufzutragende Schicht kann lediglich aus einem pulverförmigen Harz bestehen, oder es ist auch möglich ein Gemisch enthaltend das Harz, natürliche und/oder synthetische Fasern, und ggf. weitere Additive zu verwenden.

Das Pulver setzt sich dabei aus 30 bis 65 Gew%, bevorzugt 40 bis 60 Gew% Fasern, 20 bis 45 Gew%, bevorzugt 30 bis 40 Gew% Bindemittel, und 0 bis 8 Gew%, bevorzugt 0,5 bis 6 Gew% Additiv zusammen. Die natürlichen und / oder synthetischen Fasern sind bevorzugt ausgewählt aus einer Gruppe gebleichte Zellulosefasern oder organische Polymerfasern.

### Flüssiges Harz (Flüssigoverlay)

Im Falle der Verwendung von flüssigen Harz als Harzschicht beträgt die Menge des auf die Oberfläche der Trägerplatte aufgetragenen flüssigen Harzes zwischen 50 und 150 g/m², bevorzugt zwischen 60 und 100 g/m², insbesondere bevorzugt zwischen 70 und 80 g/m², wobei der Feststoffgehalt des Harzes bei ca. 65 Gew% liegt und die üblichen Hilfsstoffe wie Härter, Netzmittel usw. enthält.

Als flüssiges Harz wird ein Formaldehydharz, bevorzugt ein Harnstoffharz, ein Melaminharz oder ein Phenolharz, insbesondere bevorzugt ein Melamin-Formaldehyd-Harz, ein Melamin-Phenol-Formaldehyd-Harz oder ein Melamin-Harnstoff-Formaldehydharz verwendet.

Wie im Falle des Harzpulvers kann auch das flüssige Harz in einem Gemisch mit natürlichen und/oder synthetische Fasern, und ggf. weitere Additive verwendet werden.

### Additiv

Wie bereits oben erwähnt, kann gemäß dem vorliegenden Verfahren auf oder in die mindestens eine Harzschicht mindestens ein Additiv auf - oder eingebracht werden.

In einer bevorzugten Ausführungsform wird auf die (bevorzugt klebrige) Oberfläche der Harzschicht, z.B. der harzgetränkten Papierlage, mindestens ein Additiv aufgetragen. Das Additiv kann in flüssiger oder fester Form, insbesondere als partikelförmiger Feststoff (Staub, Pulver, Granulat), oder als Flüssigkeit oder Paste auf die Papierlage zum Beispiel mittels Sprühen, Spritzen, Gießen, Rakeln, Walzen, Streuen aufgebracht werden.

Es kann ein Additiv oder Mischungen von mehreren Additiven verwendet werden, wobei auch mehrere Additiven nacheinander auftragbar sind.

Die zum Einsatz kommenden Additive können aus folgender Gruppe ausgewählt sein: Farbstoffe (Tinte), Pigmente (z.B. Farbpigmente, Metallpigmente oder reflektierende Pigmente) Flammschutzmittel (z.B. Ammoniumpolyphosphat, Tris(tri-bromneopentyl)phosphat, Zinkborat oder Borsäurekomplexe von mehrwertigen Alkoholen), Mittel zur Erhöhung der Leitfähigkeit, UV-Stabilisatoren, Bleichmittel, Hydrophobierungsmittel oder antimikrobielle Wirkstoffe.

Mögliche antimikrobielle Wirkstoffe können mindestens ein Biozid umfassen. Voraussetzung für die Auswahl eines geeigneten Biozids ist, das dieses der EU-Verordnung Nr. 528/2012 über das Inverkehrbringen von Biozid-Produkten entspricht. Biozide können entweder nach Produktarten wie Desinfektionsmittel und Schutzmittel oder nach deren Zielorganismen (Viruzide, Bakterizide, Fungizide etc.) eingeteilt werden. Vorliegend kann das mindestens eine Biozid ausgewählt sein aus einer Gruppe umfassend Benzalkoniumchlorid, Oktylammoniumchlorid, Chitosan, Phenylphenol, Kupfersulfat, Silbernitrat, Milchsäure, Nonansäure, Natriumbenzoat, 1-[[2-(2,4-dichlorophenyl)-4-propyl-1,3-dioxolan-2-yl]methyl]-1H-1,2,4-triazole, 2-octyl-2H-isothiazol-3-one, Thiazol-4-yl-1H-benzoimidazole, 3-Iodo-2-propynylbutylcarbamat, Biphenyl-2-ol, Bronopol / Kalzium- Magnesiumoxid, Kupfer (II) oxid, 2-Pyridinthiol-1-oxid, Silberoxid, Silber-Kupfer-Zeolith. Die angeführten Wirkstoffe stammen aus Produktfamilien 2 und 9, die für antiviral wirkende Fußböden bereits genehmigt sind bzw. sich in Genehmigung befinden.

Bevorzugt ist das Additiv in dem auf der Oberfläche der Papierlage vorgesehenen Harz nicht lösbar bzw. nicht homogen lösbar. So wird gewährleistet, dass sich das Additiv nicht mit dem Harz vermischt, sondern auf der Oberfläche verbleibt und dadurch mit dem porösen Beschichtungsmaterial in Kontakt kommen und in dieses penetrieren kann.

### Trägerplatte

In einer Ausführungsform des vorliegenden Verfahrens ist die mindestens eine Trägerplatte eine Platte aus einem Holzwerkstoff, insbesondere eine Span-, mitteldichte Faser (MDF)-, hochdichte Faser (HDF)-, Oriented Strand Board (OSB)- oder Sperrholzplatte, aus Kunststoff, einem Holzwerkstoff-Kunststoff-Gemisch oder einem Verbundwerkstoff, , eine Zementfaserplatte, Gipsfaserplatte oder eine WPC-Platte (Wood Plastic Composites) oder eine SPC-Platte (Stone Plastic Composites).

Die Oberfläche des Trägermaterials kann oberflächenbehandelt sein. Auch kann die Oberfläche einer Holzwerkstoff-Trägerplatte geschliffen sein (ohne Presshaut) oder auch nicht-geschliffen sein (mit Presshaut). Im Falle einer Kunststoff-Trägerplatte kann die Oberfläche koronabehandelt sein.

### Poröses Beschichtungsmaterial

Das mindestens eine poröse Beschichtungsmaterial kann ausgewählt sein aus den folgenden Materialien: eine Furnierlage, ein Ledermaterial, Filzmaterial, Vliesmaterial und andere Stoffmaterialien. Insbesondere sind Materialien umfasst, die eine Porosität aufweisen, in der flüssiges Harz während des Verpressens aufsteigen kann und die zumindest teilweise plastisch verformbar sind.

Im Falle der Verwendung einer Furnierlage umfasst diese in einer Ausführungsform mindestens eine Lage aus Echtholzfurnier.

In einer weitergehenden Ausführungsform umfasst das mindestens eine Furnier mindestens eine Echtholz-Lage mit einer Dicke zwischen 0,2-10 mm, bevorzugt 0,5-5 mm, insbesondere bevorzugt 0,5-2 mm. Das Furnier kann einstückig von einem Stamm, zum Beispiel durch Schälen hergestellt sein. Es kann aber auch aus einzelnen Stücken zusammengesetzt sein, die zum Beispiel durch Bindemittel oder sogenannte Leimfäden miteinander verbunden sind. Das Furnier weist bevorzugt die Abmessungen der Trägerplatte auf. Das Furnier weist eine der Trägerplatte zugewandte Unterseite und eine der Trägerplatte abgewandte Oberseite auf.

Im Falle der Verwendung vom Ledermaterialien, z.B. als Dämmschicht, wird bevorzugt ein Lederfaserwerkstoff mit einer Dicke zwischen 0,5 mm und 1 mm, bevorzugt 0,75 mm verwendet.

Als Lederwerkstoff oder auch Lederfaserstoff wird hierbei ein Werkstoff aus Falzspänen z.B. Chromfalzspänen und zerkleinerten, pflanzlich gegerbten Lederresten der lederverarbeitenden Industrie, Bindemittel z.B. Naturlatex und natürlichen Fetten definiert. Der Anteil an Leder in einem Lederfaserstoff beträgt mindestens 50%. Die verarbeiteten Lederreste können unter anderem vom Rind oder auch anderen Tieren, wie zum Beispiel Pferden, stammen.

In einer weitergehenden Ausführungsform des vorliegenden Verfahrens werden die mindestens eine Trägerplatte, die mindestens eine auf der Trägerplatte angeordnete Harzschicht und das mindestens eine poröse Beschichtungsmaterial bei Temperaturen zwischen 150 und 200°C, bevorzugt zwischen 170 und 180°C bei einem Druck von 30 bis 50 kg/cm², bevorzugt 40 kg/cm² für 30-120 Sekunden, bevorzugt 60 bis 90 Sekunden verpresst.

Das vorliegende Verfahren ermöglicht somit die Bestimmung des Penetrationsgrades von Harz in ein mit einer Trägerplatte verpresstem porösen Beschichtungsmaterials mit folgenden Schichtaufbauten:
a) Holzwerkstoffplatte - harzgetränkte Papierlage (Overlay)- ggfs.- Harzpulver- Additive, poröses Beschichtungsmaterial,
b) Holzwerkstoffplatte - Harzpulver (Pulveroverlay)- Additive, poröses Beschichtungsmaterial, oder
c) Holzwerkstoffplatte - Flüssigharz (Flüssigoverlay)- Additive, poröses Beschichtungsmaterial.

Die NIR Messung der Penetrationshöhe des Harzes in das poröse Beschichtungsmaterial kann in einer Variante kontinuierlich innerhalb, d.h. online, der Produktionslinie der Werkstoffplatten bestimmt werden. In dieser online-Variante wird demnach die Penetrationshöhe im laufenden Produktionsprozess bestimmt. Dies ermöglicht eine direkte Steuerung und Eingriff in den Produktionsprozess.

In einer zweiten Ausführungsvariante des vorliegenden Verfahrens kann die Penetrationshöhe auch außerhalb (d.h. offline) der Produktionslinie der Werkstoffplatten bestimmt werden. In dieser Variante wird demnach eine fertig verpresste Werkstoffplatte aus der Produktionslinie entnommen bzw. ausgeschleust und offline z.B. in einem separaten Labor im Rahmen einer routinemäßigen Qualitätskontrolle vermessen.

In einer weiteren Variante kann die NIR-Messung sowohl online als auch offline erfolgen.

Es kann auch vorgesehen sein, dass der mindestens eine NIR-Messkopf sich quer zur Laufrichtung der mit dem porösen Beschichtungsmaterial verpressten Trägerplatten bewegt. Der NIR-Detektor kann an beliebigen Stellen in Transportrichtung der Platte installiert werden. Dabei kann der Detektor auch über die Plattenbreite traversieren bzw. bestimmte Problembereiche analysieren (z. B. im Rand- oder Mittelbereich der Platten usw.). Außerdem stehen die Messwerte sofort zur Verfügung und erlauben einen sofortigen Eingriff in den Prozess. Dies ist bei anderen Verfahren nicht ohne weiteres möglich.

Das vorliegende Verfahren wird in einer Produktionslinie umfassend mindestens einen NIR-Multimesskopf, bevorzugt mindestens zwei NIR-Multimessköpfe, und mindestens ein Steuerungssystem durchgeführt. Eine solche Produktionslinie kann eine Produktionslinie zur Herstellung von Werkstoffplatten sein. Bevorzugt wird das vorliegende Verfahren zur Bestimmung der Harzpenetration in das poröse Beschichtungsmaterial kontinuierlich und online durchgeführt.

Das Steuerungssystem der Produktionslinie umfasst mindestens eine computergestützte Auswerteeinheit (bzw. Prozessoreinheit) und eine Datenbank. In der Auswerteeinheit erfolgt der Abgleich bzw. Vergleich des für das Produkt (d.h. verpresstes poröses Beschichtungsmaterial) gemessenen NIR-Spektrums mit den für die jeweils einzelnen Parameter erstellten Kalibriermodellen. Die so bestimmten Parameterdaten werden in der Datenbank gespeichert.

Die mit dem vorliegenden spektroskopischen Verfahren bestimmten Daten können zur Steuerung der Produktionslinie verwendet werden. Die berührungslos gemessenen Parameterwerte des NIR-Multimesskopfes ("Ist-Werte") können, wie zuvor bereits beschrieben, direkt und in "real time" für die Steuerung bzw. Regelung der betreffenden Anlage verwendet werden, indem beispielsweise die gemessenen und in der Datenbank, z.B. einer relationalen Datenbank, Ist-Werte gespeichert und mit dort vorhandenen Soll-Werten dieser Parameter verglichen werden. Die sich ergebenden Differenzen werden anschließend zur Steuerung bzw. Regelung der Produktionslinie verwendet.

Für den Abgleich und die Steuerung der Produktionslinie wird ein computerimplementiertes Verfahren sowie ein Computerprogramm umfassend Befehle, die bei der Ausführung des Programms durch einen Computer diesen veranlassen das computerimplementierte Verfahren auszuführen, bereitgestellt. Das Computerprogramm ist in einer Speichereinheit des Steuerungssystems der Produktionslinie gespeichert.

Die Erfindung wird nachfolgend unter Bezugnahme auf die Figuren der Zeichnungen an einem Ausführungsbeispiel näher erläutert. Es zeigt:

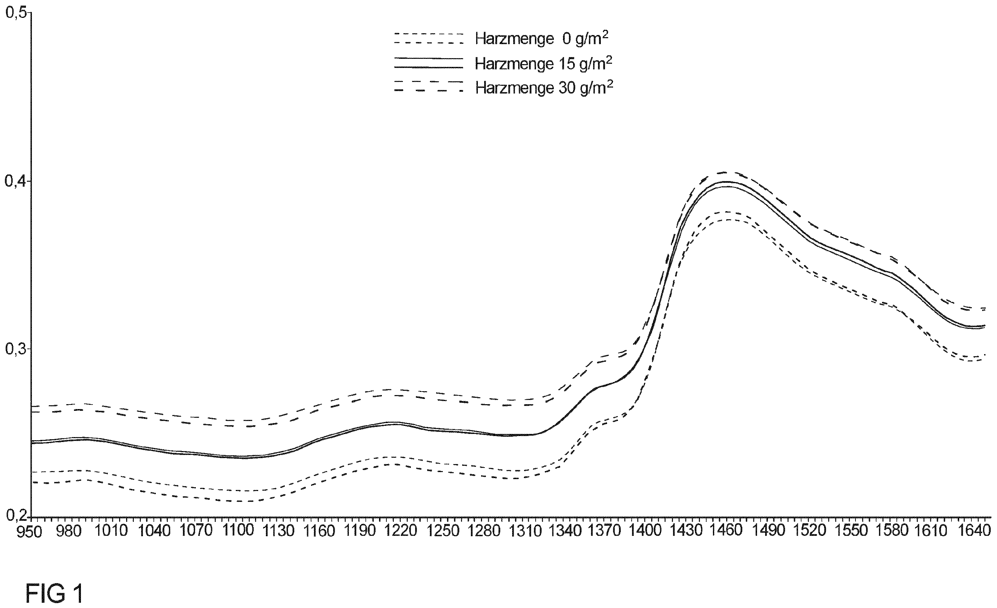

- Figur 1: ein NIR-Spektrum einer mit einer Harzschicht und Trägerplatte verpressten Furnierlage.

### Ausführungsbeispiel 1:

Drei 8 mm HDF (500 x 500 mm) wurden einseitig mit einem Overlay belegt und auf das Overlay wurde schwarze Digitaldruckfarbe in einer Menge von10 g fl./m² aufgetragen. Das Overlay hatte ein Papiergewicht von ca. 25 g/m² und einen Harzauftrag von 400%.

In das noch feuchte Overlay wurde Melaminharzpulver in Mengen von 0, 15 und 30 g/m² auf die drei Overlays aufgebracht. Die Overlays wurden im Abzug getrocknet.

Auf die Overlays wurde dann ein Eichenfurnier (Stärke: 0,5 mm) aufgelegt. Dann wurde der Aufbau in einer Laborpresse bei 180°C, einem Druck von 40 kg/cm² und einer Presszeit von 60 Sekunden verpresst. Das Furnier wurde dadurch auf eine Stärke von 0,35 mm komprimiert.

Danach wurden aus den Platten Proben geschnitten (100 x100 mm, je vier Stück). Nach dem Abkühlen wurde die Oberfläche mit einem NIR-Messkopf an vier Stellen vermessen, die durch ein Koordinatenkreuz gekennzeichnet waren und an denen später der Abrieb/Abtrag durch den Taber-Abraser erfolgt.

Anschließend wurden diese auf einem Taber-Abraser geprüft. Die Prüfung erfolgte in Anlehnung an die DIN EN 13329. Die Reibräder des Taber-Abrasers wurden mit den üblichen Schleifpapieren beklebt und auch mit den üblichen Gewichten belastet. Dann wurde jeweils nach 200 Umdrehungen visuell geprüft, ob bereits schwarze Verfärbungen im Furnier zu beobachten waren. Dann wurde mit einer Messuhr in der durch das Schleifpapier erzeugten kreisförmigen Vertiefung in den vier Kreissegmenten, die durch ein Koordinatenkreuz gebildet werden, der Abtrag in mm bestimmt und daraus der Mittelwert gebildet. Aus diesem Mittelwert wurde zusammen mit den vier anderen Proben ein Gesamtmittelwert gebildet. Von der Furnierstärke, die mit Hilfe eines Mikroskops bestimmt wurde, wurde dann der Abtrag subtrahiert und dann mit den Spektren korreliert.

Die ermittelten Werte sind in der folgenden Tabelle 1 zusammengefasst. Es ist ersichtlich, dass bei höheren Mengen (30 g/m²) von auf das Overlaypapier aufgetragenen Melaminharzpulver die mechanische Abnahme in Taber-Abraser Test geringer ausfällt, als bei 0 g/m² oder 15 g/m² an Harzpulver. Dies belegt, dass je mehr Harzpulver aufgetragen, desto mehr Harz dringt in die Furnierlage ein und desto weniger muss im Taber-Abraser Test abgetragen werden, um die schwarzen Verfärbungen in der Furnierlage zu beobachten.

Die mechanische Abnahme in Taber-Abraser Test korrespondiert dabei mit der durch das NIR-Verfahren ermittelten Abnahme, so dass das NIR-Verfahren einen Nachweis der Penetrationshöhe des Harzes in der Furnierlage zulässt.

**Tabelle 1**

| Menge an aufgetragenem Melaminharz | Abnahme in mm NIR | Abnahme in mm Taber.Abraser | Differenz in mm |
|---|---|---|---|
| Nullprobe | 0,25 | 0,28 | 0,03 |
| 15 g Melaminharz/m² | 0,12 | 0,15 | 0,03 |
| 30 g Melaminharz/m² | 0,07 | 0,07 | 0 |

Der Messkopf zur Bestimmung der Harzpenetration wird unmittelbar hinter der verwendeten Presse installiert. Durch eine automatisierte Verschiebemöglichkeit kann der Messkopf unterschiedliche Bereiche eine mit Furnier beschichteten Platte analysieren oder er kann über die Platte traversieren. Dadurch ist sichergestellt, dass auch Bereiche, die üblicherweise wegen unterschiedlichen Pressbedingungen problematisch sein können (z. B. Plattenränder) analysiert werden.

Bei einer nicht adäquaten Penetration des Harzes in das Furnier kann durch Veränderung der Presstemperatur und/oder der Presszeit ein verbesserter Harzfluss erreicht werden. Dabei werden die beiden Parameter gegenläufig verändert. Bei einer Reduktion der Presstemperatur wird die Presszeit verlängert. Beispielsweise wird bei einer Reduzierung der Presstemperatur um 10°C die Presszeit um 10 bis 20 Sekunden verlängert.

### Ausführungsbeispiel 2:

Zur Prüfung der Genauigkeit der Kalibration wurde statt einer Beschichtung eines HWS mit einem Furnier eine Beschichtung mit einem Leder durchgeführt. Dabei wurde zunächst mit Hilfe eines NIR-Messgerätes ein Spektrum des eingesetzten Leders erstellt, um zu prüfen, ob der Melaminpeak bei ca. 1500 nm durch Peaks des Leders überlagert wird. Was sich nicht bestätigt hat.

Auf eine 8 mm HDF (500 x 500 mm) wurde einseitig mit einem Overlay belegt. Das Overlay hatte ein Papiergewicht von ca. 25 g/m² und einen Harzauftrag von 400%.

Auf das Overlay wurde dann ein braunes Leder (Stärke: 0,75 mm) aufgelegt. Dann wurde der Aufbau in einer Laborpresse bei 180°C, einem Druck von 40 kg/cm² und einer Presszeit von 60 Sekunden verpresst. Das Leder wurde dadurch auf eine Stärke von 0,45 mm komprimiert.

Danach wurden aus der Platte Proben geschnitten (100 x100 mm, je vier Stück). Nach dem Abkühlen wurde die Oberfläche mit einem NIR-Messkopf an vier Stellen vermessen, die durch ein Koordinatenkreuz gekennzeichnet waren.

Die Messung mit dem NIR-Messgerät ergab eine Eindringtiefe von 0,35 mm. Dies wurde anschliessend mit dem Taber-Abraser überprüft. Dabei wurde ein Wert von 0,35 ermittelt.

Andere poröse Beschichtungsmaterialien wie Stoff, Filz, Vlies usw. können ebenfalls über dieses Verfahren vermessen werden.

## Patentansprüche

1. Verfahren zur Bestimmung der Harzpenetration in mindestens ein poröses Beschichtungsmaterial, welches mit mindestens einer Trägerplatte und mindestens einer auf der Trägerplatte angeordneten Harzschicht verpresst ist, wobei während des Pressvorganges das Harz in das mindestens eine poröse Beschichtungsmaterial penetriert bzw. aufsteigt,
umfassend die Schritte
- Aufnahme von mindestens einem NIR-Spektrum von mehreren Referenzproben mit jeweils unterschiedlichem Werten für die Harzpenetration in ein poröses Beschichtungsmaterial unter Verwendung von mindestens einem NIR-Messkopf in einem Wellenlängenbereich zwischen 500 nm und 2500 nm, bevorzugt zwischen 700 nm und 2000 nm, insbesondere bevorzugt zwischen 900 nm und 1700 nm, und besonders vorteilhaft zwischen 1450 nm und 1550 nm; wobei die Referenzproben gleichartig sind zu der zu vermessenden Probe,
- Bestimmung der Harzpenetration in das poröse Beschichtungsmaterial der genannten Referenzproben mittels eines mechanischen Abtrages der porösen Materialoberfläche;
- Zuordnung der mittels mechanischen Abtrages bestimmten Harzpenetration zu den aufgenommenen NIR-Spektren der genannten Referenzproben; und
- Erstellung eines Kalibriermodells für den Zusammenhang zwischen den spektralen Daten der NIR-Spektren und den dazugehörigen Harzpenetrationen der Referenzproben mittels einer multivariaten Datenanalyse;
- Verpressen von mindestens einem porösen Beschichtungsmaterial mit mindestens einer Trägerplatte und mindestens einer auf der Trägerplatte angeordneten Harzschicht,
- Aufnehmen von mindestens einem NIR-Spektrum des mit der Trägerplatte und der Harzschicht verpressten porösen Beschichtungsmaterials unter Verwendung des mindestens einen NIR-Messkopfes in einem Wellenlängenbereich zwischen 500 nm und 2500 nm, bevorzugt zwischen 700 nm und 2000 nm, insbesondere bevorzugt zwischen 900 nm und 1700 nm und besonders vorteilhaft zwischen 1450 nm und 1550 nm; und
- Bestimmen der Harzpenetration in das mindestens eine poröse Beschichtungsmaterial durch Vergleich des für das poröse Beschichtungsmaterialaufgenommenen NIR-Spektrums mit dem erstellten Kalibriermodell,
wobei die Bestimmung der Harzpenetration berührungslos als Echtzeit (real time) Messung durchgeführt wird.

2. Verfahren nach Anspruch 1, **dadurch gekennzeichnet, dass** die mindestens eine Harzschicht eine harzgetränkten Papierlage, ein harzhaltiges Pulver oder eine harzhaltige Flüssigkeit umfasst.

3. Verfahren nach Anspruch 2, **dadurch gekennzeichnet, dass** Im Falle der Verwendung von pulverförmigem Harz als Harzschicht die Menge des auf die Oberfläche der Trägerplatte aufgetragenen pulverförmigen Harzes 50-150g/m², bevorzugt 60-100 g/m², insbesondere bevorzugt 70-80 g/m² beträgt.

4. Verfahren nach Anspruch 2 oder 3, **dadurch gekennzeichnet, dass** das pulverförmige Harz eine Streudichte von 0,5 bis 1,5 kg/l, bevorzugt 0,8 bis 1,0 kg/l und einer mittlere Partikelgröße von 10 bis 50 µm, bevorzugt 20 bis 30 µm, insbesondere bevorzugt von 25 µm aufweist.

5. Verfahren nach einem der vorhergehenden Ansprüche, **dadurch gekennzeichnet, dass** auf die mindestens eine Harzschicht mindestens ein Additiv aufgebracht wird.

6. Verfahren nach Anspruch 5, **dadurch gekennzeichnet, dass** das mindestens eine Additiv ausgewählt ist aus folgender Gruppe umfassend Farbstoffe (z.B.Tinte), Pigmente (z.B. Farbpigmente, Metallpigmente oder reflektierende Pigmente) Flammschutzmittel (z.B. Ammoniumpolyphosphat, Tris(tri-bromneopentyl)phosphat, Zinkborat oder Borsäurekomplexe von mehrwertigen Alkoholen), Mittel zur Erhöhung der Leitfähigkeit, UV-Stabilisatoren, Bleichmittel, Hydrophobierungsmittel oder antimikrobielle Wirkstoffe.

7. Verfahren nach Anspruch 5-6, **dadurch gekennzeichnet, dass** das mindestens eine Additiv ein Farbstoff ist.

8. Verfahren nach einem der vorhergehenden Ansprüche, **dadurch gekennzeichnet, dass** die mindestens eine Trägerplatte eine Platte aus einem Holzwerkstoff, insbesondere eine Span-, mitteldichte Faser (MDF)-, hochdichte Faser (HDF)-, Oriented Strand Board (OSB)- oder Sperrholzplatte, aus Kunststoff, einem Holzwerkstoff-Kunststoff-Gemisch oder einem Verbundwerkstoff, , eine Zementfaserplatte, Gipsfaserplatte oder eine WPC-Platte (Wood Plastic Composites) oder eine SPC-Platte (Stone Plastic Composites) ist.

9. Verfahren nach einem der vorhergehenden Ansprüche, **dadurch gekennzeichnet, dass** das mindestens eine poröse Beschichtungsmaterial mindestens eine Furnierlage, ein Ledermaterial, Filzmaterial, Vliesmaterial und/oder solche Materialien umfasst, die eine Porosität aufweisen, in der flüssiges Harz während des Verpressens aufsteigen kann und die zumindest teilweise plastisch verformbar sind.

10. Verfahren nach einem der vorhergehenden Ansprüche, **dadurch gekennzeichnet, dass** die mindestens eine Trägerplatte, die mindestens eine auf der Trägerplatte angeordnete Harzschicht und das mindestens eine poröse Beschichtungsmaterial bei Temperaturen zwischen 150 und 200°C, bevorzugt zwischen 170 und 180°C bei einem Druck von 30 bis 50 kg/cm², bevorzugt 40 kg/cm² für 30-120 Sekunden, bevorzugt 60 bis 90 Sekunden verpresst werden.

11. Verfahren nach einem der vorhergehenden Ansprüche, **dadurch gekennzeichnet, dass** für die Erstellung des Kalibriermodells spektrale Daten aus dem gesamten aufgenommenen Spektralbereich verwendet werden.

12. Verfahren nach einem der vorhergehenden Ansprüche, **dadurch gekennzeichnet, dass** für die Erstellung des Kalibriermodells spektrale Daten aus dem NIR-Spektralbereich zwischen 1450 nm und 1550 nm verwendet werden, die mittels geeigneter mathematischer Methoden vorbehandelt werden und anschließend der multivariaten Datenanalyse zugeführt werden.

13. Verfahren nach einem der vorhergehenden Ansprüche, **dadurch gekennzeichnet, dass** die Bestimmung der Harzpenetration in das poröse Beschichtungsmateriale kontinuierlich und online in einer Produktionslinie zur Herstellung von Werkstoffplatten umfassend mindestens einen NIR-Multimesskopf, mindestens eine computergestützte Auswerteeinheit und eine Datenbankerfolgt.

14. Verfahren nach Anspruch 13, **dadurch gekennzeichnet, dass** die gemessenen Daten der NIR-Spektren zur Bestimmung der Harzpenetration als Ist-Werte direkt und in "real time" für die Steuerung bzw. Regelung der Produktionslinie verwendet werden, wobei die gemessenen Ist-Werte mit in der Datenbank gespeicherten Soll-Werten verglichen werden, und die sich ergebenden Differenzen anschließend zur Steuerung bzw. Regelung der Produktionslinie verwendet werden.

## Claims

1. Method for determining the resin penetration into at least one porous coating material which is pressed with at least one carrier board and at least one resin layer arranged on the carrier board, wherein during the pressing process the resin penetrates or rises into the at least one porous coating material,
comprising the steps
- Recording of at least one NIR spectrum of several reference samples each having different values for resin penetration into a porous coating material using at least one NIR measuring head in a wavelength range between 500 nm and 2500 nm, preferably between 700 nm and 2000 nm, more preferably between 900 nm and 1700 nm, and particularly advantageously between 1450 nm and 1550 nm; wherein the reference samples are similar to the sample to be measured;
- Determining the resin penetration into the porous coating material of the mentioned reference samples by means of a mechanical removal of the porous material surface;
- Correlating the resin penetration determined by mechanical removal with the recorded NIR spectra of said reference samples; and
- Creating a calibration model for the correlation between the spectral data of the NIR spectra and the corresponding resin penetrations of the reference samples by means of multivariate data analysis;
- Pressing of at least one porous coating material with at least one carrier board and at least one resin layer arranged on the carrier board,
- Recording at least one NIR spectrum of the porous coating material pressed with the carrier board and the resin layer using the at least one NIR measuring head in a wavelength range between 500 nm and 2500 nm, preferably between 700 nm and 2000 nm, in particular preferably between 900 nm and 1700 nm and particularly advantageously between 1450 nm and 1550 nm; and
- Determining the resin penetration into the at least one porous coating material by comparing the NIR spectrum recorded for the porous coating material with the calibration model created,
Wherein the determination of the resin penetration is carried out without contact in real-time.

2. Method according to claim 1, **characterized in that** the at least one resin layer comprises a resin-impregnated paper layer, a resin-containing powder or a resin-containing liquid.

3. Method according to claim 2, **characterized in that**, in case of using powdered resin as resin layer, the amount of powdered resin applied to the surface of the carrier board is 50-150g/m², preferably 60-100 g/m², more preferably 70-80 g/m².

4. Method according to claim 2 or3, **characterized in that** the powdered resin has a scattering density of 0.5 to 1.5 kg/l, preferably 0.8 to 1.0 kg/l, and an average particle size of 10 to 50 µm, preferably 20 to 30 µm, more preferably 25 µm.

5. Method according to one of the preceding claims, **characterized in that** at least one additive is applied to the at least one resin layer.

6. Method according to claim 5, **characterized in that** the at least one additive is selected from the following group comprising dyes (for example ink), pigments (for example colour pigments, metallic pigments or reflective pigments), flame retardants (for example ammonium polyphosphate, tris(tri-bromo neopentyl) phosphate, zinc borate or boric acid complexes of polyhydric alcohols), agents for increasing conductivity, UV stabilizers, bleaching agents, hydrophobing agents or antimicrobial agents.

7. Method according to claim 5-6, **characterized in that** the at least one additive is a dye.

8. Method according to one of the preceding claims, **characterized in that** the at least one carrier board is a board made of a wood material, in particular a particle board, medium-density fibre board (MDF), high-density fibre board (HDF), oriented strand board (OSB) or plywood board, of plastic, a wood material-plastic mixture or a composite material, a cement fibre board, gypsum fibre board or a WPC-board (wood plastic composites) or a SPC board (stone plastic composites).

9. Method according to one of the preceding claims, **characterized in that** the at least one porous coating material comprises at least one veneer layer, leather material, felt material, non-woven material and/or such materials which have a porosity in which liquid resin can rise during pressing and which are at least partially plastically deformable.

10. Method according to one of the preceding claims, **characterized in that** the at least one carrier board, the at least one resin layer disposed on the carrier board and the at least one porous coating material are compressed at temperatures between 150 and 200°C, preferably between 170 and 180°C, at a pressure of 30 to 50 kg/cm², preferably 40 kg/cm² for 30-120 seconds, preferably 60 to 90 seconds.

11. Method according to one of the preceding claims, **characterized in that** spectral data from the entire recorded spectral range are used to create the calibration model.

12. Method according to one of the preceding claims, **characterized in that** spectral data from the NIR spectral range between 1450 nm and 1550 nm are used for the creating the calibration model, which are pre-treated by means of suitable mathematical methods and are subsequently fed to the multivariate data analysis.

13. Method according to one of the preceding claims, **characterized in that** the determination of the resin penetration into the porous coating material is carried out continuously and online in a production line for manufacturing material boards comprising at least one NIR multimeter head, at least one computer-aided evaluation unit and a database.

14. Method according to claim 13, **characterized in that**, the data determined are used directly and in "real time" for the control or regulation of the production line, wherein the actual values measured are stored in the database are compared with target values of these parameters existing there, and the resulting differences are then used to control or regulate the production line.

## Revendications

1. Procédé de détermination de la pénétration de la résine dans au moins un matériau de revêtement poreux, qui est pressé avec au moins une plaque de support et au moins une couche de résine disposée sur la plaque de support, la résine pénétrant ou remontant dans au moins un matériau de revêtement poreux pendant le processus de pressage,
comprenant les étapes suivantes
- Enregistrement d'au moins un spectre NIR à partir de plusieurs échantillons de référence, chacun ayant des valeurs de pénétration de résine différentes, dans un matériau de revêtement poreux à l'aide d'au moins une tête de mesure NIR dans une plage de longueurs d'onde comprise entre 500 nm et 2500 nm, de préférence entre 700 nm et 2000 nm, en particulier de préférence entre 900 nm et 1700 nm, et de manière particulièrement avantageuse entre 1450 nm et 1550 nm ; dans lequel les échantillons de référence sont similaires à l'échantillon à mesurer,
- Détermination de la pénétration de la résine dans le matériau de revêtement poreux desdits échantillons de référence au moyen d'une abrasion mécanique de la surface du matériau poreux ;
- Attribution de la pénétration de la résine, déterminée par abrasion mécanique, aux spectres NIR enregistrés desdits échantillons de référence ; et
- Établissement d'un modèle d'étalonnage pour la relation entre les données spectrales des spectres NIR et les pénétrations de résine correspondantes des échantillons de référence au moyen d'une analyse de données multivariée ;
- Compression d'au moins un matériau de revêtement poreux avec au moins une plaque de support et au moins une couche de résine disposée sur la plaque de support,
- Enregistrement d'au moins un spectre NIR du matériau de revêtement poreux pressé avec la plaque de support et la couche de résine en utilisant au moins une tête de mesure NIR dans une plage de longueurs d'onde entre 500 nm et 2500 nm, de préférence entre 700 nm et 2000 nm, en particulier de préférence entre 900 nm et 1700 nm et de manière particulièrement avantageuse entre 1450 nm et 1550 nm ; et
- Détermination de la pénétration de la résine dans au moins un matériau de revêtement poreux en comparant le spectre NIR enregistré pour le matériau de revêtement poreux avec le modèle d'étalonnage établi,
la détermination de la pénétration de la résine étant effectuée sans contact sous la forme d'une mesure en temps réel (real time).

2. Procédé selon la revendication 1, **caractérisé en ce qu'**au moins une couche de résine comprend une couche de papier imprégnée de résine, une poudre résineuse ou un liquide résineux.

3. Procédé selon la revendication 2, **caractérisé en ce que**, en cas d'utilisation de la résine en poudre comme couche de résine, la quantité de résine en poudre appliquée sur la surface de la plaque de support est de 50 à 150 g/m², de préférence de 60 à 100 g/m², en particulier de préférence de 70 à 80 g/m².

4. Procédé selon la revendication 2 ou 3, **caractérisé en ce que** la résine en poudre présente une densité de diffusion de 0,5 à 1,5 kg/l, de préférence de 0,8 à 1,0 kg/l et une taille moyenne de particules de 10 à 50 µm, de préférence de 20 à 30 µm, en particulier de préférence de 25 µm.

5. Procédé selon l'une quelconque des revendications précédentes, **caractérisé en ce qu'**au moins un additif est appliqué sur au moins une couche de résine.

6. Procédé selon la revendication 5, **caractérisé en ce qu'**au moins un additif est choisi dans le groupe suivant, comprenant des colorants (par exemple, l'encre), des pigments (par exemple, des pigments de couleur, des pigments métalliques ou des pigments réfléchissants), des retardateurs de flamme (par exemple, polyphosphate d'ammonium, tris(tri-bromonéopentyl)phosphate, du borate de zinc ou complexes d'acide borique d'alcools polyhydriques), des agents d'augmentation de la conductivité, des stabilisants UV, des agents de blanchiment, des agents hydrophobes ou des agents antimicrobiens.

7. Procédé selon la revendication 5-6, **caractérisé en ce qu'**au moins un additif est un colorant.

8. Procédé selon l'une quelconque des revendications précédentes, **caractérisé en ce qu'**au moins une plaque de support est un panneau en matériau à base de bois, en particulier des panneaux d'aggloméré, de fibres à densité moyenne (MDF), de fibres à haute densité (HDF), des panneaux à copeaux orientés (OSB) ou des panneaux de contreplaqué, en plastique, en un mélange de matériau à base de bois et de matière plastique ou en matériau composite, en panneaux de fibres de ciment, en panneaux de fibres de gypse ou en panneaux WPC (composites bois-plastique) ou en panneaux SPC (composites pierre-plastique).

9. Procédé selon l'une quelconque des revendications précédentes, **caractérisé en ce qu'**au moins un matériau de revêtement poreux comprend au moins une couche de placage, un matériau en cuir, un matériau en feutre, un matériau non tissé et/ou de tels matériaux présentant une porosité dans laquelle la résine liquide peut remonter pendant le pressage et qui est au moins partiellement déformable plastiquement.

10. Procédé selon l'une quelconque des revendications précédentes, **caractérisé en ce qu'**au moins une plaque de support, la ou les couches de résine disposées sur la plaque de support et au moins un matériau de revêtement poreux sont comprimés à des températures comprises entre 150 et 200° C, de préférence entre 170 et 180° C à une pression de 30 à 50 kg/cm², de préférence 40 kg/cm² pendant 30 à 120 secondes, de préférence pendant 60 à 90 secondes.

11. Procédé selon l'une quelconque des revendications précédentes, **caractérisé en ce que** les données spectrales de l'ensemble de la gamme spectrale enregistrée sont utilisées pour la préparation du modèle d'étalonnage.

12. Procédé selon l'une quelconque des revendications précédentes, **caractérisé en ce que** des données spectrales de la gamme spectrale NIR comprise entre 1450 nm et 1550 nm sont utilisées pour la préparation du modèle d'étalonnage, qui sont prétraitées par des méthodes mathématiques appropriées et sont ensuite introduites dans l'analyse de données multivariées.

13. Procédé selon l'une quelconque des revendications précédentes, **caractérisé en ce que** la détermination de la pénétration de la résine dans le matériau de revêtement poreux est effectuée en continu et en ligne dans une chaîne de production pour la fabrication de plaques comprenant au moins une tête de mesure multiple NIR, au moins une unité d'évaluation assistée par ordinateur et une base de données.

14. Procédé selon la revendication 13, **caractérisé en ce que** les données spécifiées sont utilisées directement et en temps réel pour le contrôle ou la régulation de la chaîne de production, les valeurs mesurées étant stockées dans la base de données et comparées aux valeurs cibles de ces paramètres qui y sont disponibles, et les différences résultantes sont ensuite utilisées pour le contrôle ou la régulation de la chaîne de production.
